# EUROPEAN PATENT APPLICATION

(11) **EP 4 206 266 A1**
(43) Date of publication of application: **05.07.2023**
(21) Application number: 21306952.9
(22) Date of filing: 29.12.2021
(51) Int. Cl.: C08J 11/12, C07C 67/333, C07C 69/54, C10B 53/07, C10B 57/06, C10G 1/10, B01D 5/00, C10B 47/00

(54) **DEPOLYMERIZATION METHOD AND SYSTEM**

(71) Applicant: ARKEMA FRANCE, 92700 Colombes (FR); Japan Steel Works Europe GmbH, 40217 Düsseldorf (DE); Heathland B.V., 3565 AR Utrecht (NL)
(72) Inventor: DUBOIS, Jean-Luc, 92700 COLOMBES (FR); TOJO, Makoto, 40217 DÜSSELDORF (DE); VAN DER HEIJDEN, Simon, 3402SE IJSSELSTEIN (NL); VERBEEK, Erik, 7512EE ENSCHEDE (NL); HEESINK, Bert, 7512EE ENSCHEDE (NL)
(74) Representative: Lavoix

(57) **Abstract**

The depolymerization method is provided for recovering a monomer from a polymer and comprises the successive steps of pyrolyzing a feed material containing the polymer into a pyrolysis reactor so as to generate a gas stream, directing the gas stream from the pyrolysis reactor to a separator configured for removing impurities from the gas stream and directing the gas stream from the separator to a condenser for condensing the monomer contained in the gas stream. The temperature of the gas stream in the separator is maintained equal or higher than the boiling temperature of the monomer.

## Description

The project leading to this application has received funding from the European Union's Horizon 2020 research and innovation program under grant agreement No 820687.

The present invention relates to the field of depolymerization for recovering a monomer from a feed material containing a polymer from which the monomer is a constituent.

It relates more particularly to a thermal depolymerization method comprising the pyrolysis of the feed material, i.e. the heating of the feed material such as to vaporize the feed material at least in part, in a substantial absence of oxygen. The pyrolysis of the feed material generates a gas stream including vapor of the monomer.

A problem arising in such a depolymerization method is that the gas stream generated by the pyrolysis may contain impurities such as dust, mist droplets, remaining polymer chains, oligomers and/or contaminants, in addition to the vaporized monomer.

Such impurities may alter components of the depolymerization system located downstream the pyrolysis reactor, thus negatively impacting the productivity, or contaminate the monomer, retrieved e.g. as crude monomer or oil monomer, obtained from the vaporized monomer.

JP3210323 and DE3146194 each disclose a depolymerization method for recovering a monomer from a polymer comprising the steps of pyrolizing a feed material containing the polymer into a pyrolysis reactor such as to generate a gas stream containing monomer vapor and passing the gas stream through a separator such as to remove impurities from the gas stream.

WO2016/030460 discloses a depolymerization method for recovering a monomer from a polymer comprising the steps of pyrolizing a feed material containing the polymer into a pyrolysis reactor such as to generate a gas stream containing monomer vapor and passing the gas stream through a contactor containing perforated plates arranged for flow of long chain components back to the pyrolysis reactor.

US10731080 discloses a depolymerization method for recovering a monomer from a polymer comprising the steps of pyrolizing a waste material containing the polymer to generate a gas stream, quenching the gas stream output from the pyrolysis reactor in a quenching apparatus for removing oligomers from the gas stream and routing the oligomers back to the pyrolysis reactor.

One of the aims of the invention is to propose a depolymerization method that allows producing monomer, in particular crude monomer or pyrolysis oil, with a satisfying quality and with a satisfying productivity.

To the end, the invention proposes a depolymerization method for recovering a monomer from a polymer, the depolymerization method comprising the successive steps of:
- pyrolyzing a feed material containing the polymer into a pyrolysis reactor so as to generate a gas stream;
- directing the gas stream from the pyrolysis reactor to a separator configured for removing impurities from the gas stream;
- directing the gas stream from the separator to a condenser for condensing the monomer contained in the gas stream;
wherein the temperature of the gas stream in the separator is maintained equal or higher than the boiling temperature of the monomer.

Maintaining the temperature of the gas stream inside the separator at or above the boiling temperature of the monomer allows vaporization of any, or most, monomer that would condense inside the separator.

Preferably, the temperature should be low enough to promote the condensation into the separator of any dimer or trimer present in the gas stream or that could form again from the monomer in the separator.

In a particular embodiment, it may be possible to trap mist droplets inside the separator for improved efficiency, without clogging the separator. This would enable to collect mist droplets that are generated around fine dust particles transported in the gas stream entering the separator.

According to specific embodiments, the depolymerization method comprises one or several of the following optional features, taken in isolation or in any technically feasible combination:
- the temperature of the gas stream in the separator is lower than the boiling temperature of dimer of the monomer and/or lower than the boiling temperature of trimer of the monomer at least in a downstream section of the separator;
- the largest cross-section of the internal flow passage of the separator has an area that is at least 0.001 times the mass flow rate of the gas stream entering the separator in operation, the area being expressed in square meters (m²) and the mass flow rate being expressed in kilograms per hour (kg/h);
- the area of the largest cross-section of the internal flow passage in the separator is at least ten times the area of the smallest cross-section in the separator;
- a linear velocity of the gas stream in the separator is lower than a maximum linear velocity;
- the maximum linear velocity is of 0.50 m/s, in particular 0.15 m/s, still in particular of 0.10 m/s;
- the area of the largest cross-section of an internal flow passage of the separator is chosen as a function of the maximum feed rate of the pyrolysis reactor, the depolymerization temperature of the polymer and the molar weight of the polymer such that the linear velocity of the gas stream in the downstream section of the inlet duct is lower than the maximum linear velocity at the maximum feed rate of the pyrolysis reactor;
- the residence time of the gas stream in the separator is comprised between 1 s and 30 s, preferably between 5 s and 25 s, still preferably between 10 s and 20 s;
- the method comprises spraying liquid monomer retrieved from the condenser inside the separator, the liquid monomer being sprayed into the gas stream;
- the cold monomer is sprayed into the separator such that the mass rate of cold monomer sprayed into the separator is equal or inferior to 93%, still preferably equal or inferior to 83%, still preferably equal of inferior to 73%, still preferably equal or inferior to 63%, still preferably equal or inferior to 53%;
- liquid monomer is sprayed into the separator such that the mass rate of cold monomer sprayed into the separator is comprised equal or superior to 10%, still preferably equal or superior to 20%, preferably equal or superior to 30%, still preferably equal or superior to 40%, still preferably equal or superior to 50%;
- the gas stream is slowed down progressively inside the separator;
- the cross section of the internal flow passage of the separator increases progressively when considering the direction of flow of the gas stream;
- the separator comprises a separation chamber extending along a central axis, the separator being configured for the gas stream to flow circularly in the separation chamber;
- the separator has an inlet duct for receiving the gas stream generated by the pyrolysis reactor, the inlet duct opening into the separation chamber;
- the inlet duct extends around the separation chamber;
- the method comprises coalescing mist droplets present in the gas stream by providing an internal demisting device inside the separator.

The invention also relates to a depolymerization system configured for implementing a depolymerization method as in any one of the preceding claim for recovering a monomer from a polymer, the depolymerization system comprising:
- a pyrolysis reactor for pyrolyzing a feed material containing the polymer so as to generate a gas stream from the feed material;
- a separator for receiving the gas stream from the pyrolysis reactor and removing impurities from the gas stream ;
- a condenser for receiving the gas stream from the separator for condensing the monomer contained in the gas stream;
wherein the depolymerization system is configured such that in operation the temperature of the gas stream in the separator is maintained equal to or higher than the boiling temperature of the monomer.

The invention and its advantages will be better understood upon reading the following description given solely by way of non-limiting example and with reference to the appended drawings in which:
- Figure 1 is a schematic diagram illustrating a depolymerization system for implementing a depolymerization method for recovering a monomer from a feed material containing a polymer from which the monomer is a constituent;
- Figure 2 is a diagrammatic side view of a separator that may be used in the depolymerization system of Figure 1;
- Figure 3 is a diagrammatic top view of an alternative separator of Figure 2;
- Figure 4 is a diagrammatic exploded side view of the separator of Figure 3;
- Figure 5 is a diagrammatic side view of a separator;
- Figure 6 is a diagrammatic side view of a separator;
- Figure 7 is a diagrammatic top view of a separator provided with an inlet collector;
- Figure 8 is a diagrammatic side view of the inlet collector;
- Figure 9 is a diagrammatic exploded view of a separator.

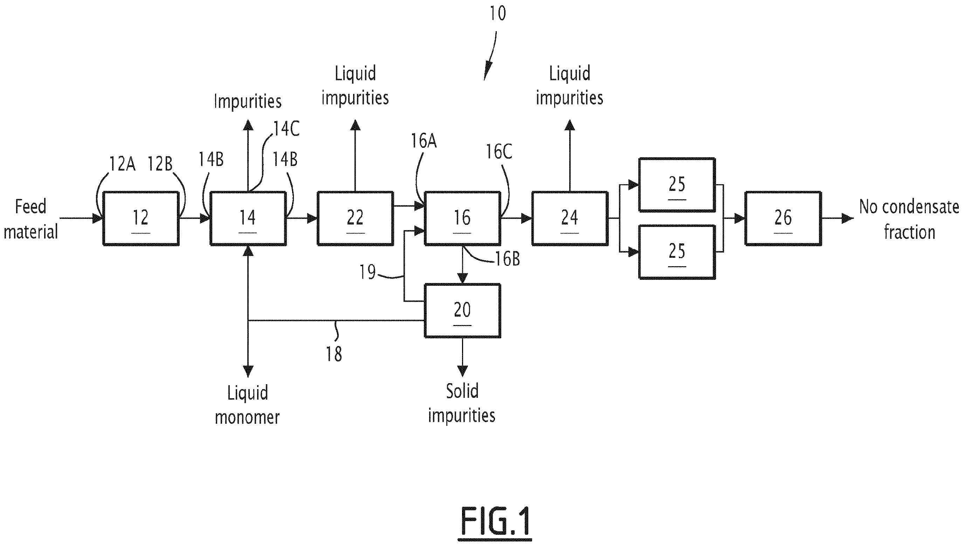

The depolymerization system 10 illustrated on Figure 1 is configured for implementing a depolymerization method for recovering a monomer from a feed material containing a polymer from which the monomer is a constituent.

The depolymerization method comprises the successive steps of pyrolizing the feed material in a pyrolysis reactor 12 configured for generating a gas stream from a feed material containing the polymer, the gas stream containing the monomer which is a constituent of the polymer, the monomer being in gaseous state, directing the gas stream from the pyrolysis reactor 12 to a separator 14 configured for removing impurities from the gas stream and directing the gas stream from the separator 14 to a condenser 16 configured for condensing the monomer in gaseous state contained in the gas stream into monomer in liquid state.

During implementation of the depolymerization method, the pyrolysis is operated such that the gas stream provided to the separator 14 contains the monomer in gaseous state, which also referred to as "gaseous monomer" in the following.

During implementation of the depolymerization method, the condensate provided by the condenser 16 contains the monomer, or pyrolysis oil, in liquid state also referred to as "liquid monomer" or "cold monomer" in the following.

The depolymerization system 10 comprises the pyrolysis reactor 12, the separator 14 and the condenser 16 fluidly connected in series in this order.

Other components of the depolymerization system 10 may optionally be incorporated between the pyrolysis reactor 12 and the separator 14 or between the separator 14 and the condenser 16 or downstream the condenser 16 as this will be detailed below.

The pyrolysis reactor 12 comprises a reactor inlet 12A for receiving the feed material and a reactor outlet 12B for providing the gas stream resulting from the pyrolysis of the feed material.

The pyrolysis reactor 12 is configured for heating the feed material received at the reactor inlet 12A at high temperature such as to vaporize the feed material to generate the gas stream provided at the reactor outlet 12B.

Optionally, the pyrolysis reactor 12 is configured for mechanically processing the feed material. Mechanically processing of the feed material in addition to heating the feed material promotes generating the gas stream.

Mechanically processing the feed material in the pyrolysis reactor 12 may for example include stirring and/or extruding the feed material. Hence, the pyrolysis reactor 12 is for example configured for stirring the feed material and/or extruding the feed material.

The pyrolysis reactor 12 is for example configured as a heating screw extruder, in particular a heating twin-screw extruder.

A heating screw extruder comprises a tubular barrel, at least one screw extending inside the barrel with being driven in rotation for extruding the feed material along the tubular chamber, and a heating system for heating the feed material contained in the barrel. A heating twin-screw extruder is a heating screw extruder comprising two screws extending side-by-side in the barrel, the threads of the screw being preferably intermeshed, the screws being either co-rotating (rotating in the same direction about their respective axes) or counter-rotating (rotating in opposed directions about their respective axes).

The separator 14 comprises a separator gas inlet 14A for receiving the gas stream generated by the pyrolysis reactor 12 and a separator gas outlet 14B for providing the gas stream to be directed to the condenser 16.

The separator 14 has an internal flow passage for the gas stream from the separator gas inlet 14A to the separator gas outlet 14B. The internal flow passage has an upstream section adjacent the separator gas inlet 14A and a downstream section adjacent the separator gas outlet 14B.

The internal flow passage has a cross-section taken substantially perpendicularly to the flow of gas inside the internal flow passage. The cross-section has an area (i.e. the measure of the extent of the cross-section).

The area of the cross-section of the internal flow passage of the separator 14 may be constant along the internal flow passage or vary along the internal flow passage.

In the following, the "largest cross-section" of the internal flow passage of the separator 14 refers to the section of the internal flow passage of the separator 14 having the cross-section that as the largest area.

Optionally, the separator 14 comprises an impurities outlet 14C for collecting impurities separated from the gas stream in the separator 14.

The condenser 16 comprises a condenser inlet 16A for receiving the gas stream provided by the separator 14 and a condenser outlet 16B for providing the condensate, i.e. the products that have condensed in the condenser 16.

The condenser 16 is configured for condensing the gas stream received at the condenser inlet 16A and provide a condensate at the condensate outlet 16B.

The condenser 16 optionally comprises a gas outlet 16C for providing non-condensate products that pass through the condenser 16 without condensing.

The depolymerization method is implemented such that the gas stream in the separator 14 is at a temperature that is equal to or higher than the boiling temperature of the monomer.

The gas stream produced by the pyrolysis travels along the inlet flow passage of the separator 14 with cooling progressively. The temperature of the gas stream in the separator 14 is the lowest in the downstream section of the separator 14.

Hence, in other words, the depolymerization method is implemented such that the temperature of the gas stream in the downstream section of the separator 14 is equal to or higher than the boiling temperature of the monomer.

This will promote evaporation of any liquid monomer present in the gas stream into gaseous monomer and this will prevent polymerization of the monomer.

The gas stream may contain dimer or trimer, i.e. chains of two monomers or three monomers. Such dimer or trimer may be due to incomplete depolymerization during pyrolysis or to polymerization of the monomer.

Generally, the boiling temperature of dimer and trimer of a monomer is strictly higher than the boiling temperature of the monomer.

Preferably, the depolymerization method is implemented such that the temperature of the gas stream in the downstream section of the separator 14 is at a temperature that is strictly inferior to the boiling temperature of the dimer of the monomer and/or inferior to the boiling temperature of the trimer of the monomer.

This will cause most of the dimer and/or trimer present in the gas stream to condensate in the separator 14 thus retaining dimer and trimer in the separator 14 and limiting or avoiding them to flow to the condenser 16.

The depolymerisation method is for example implemented with polymethyl methacrylate (PMMA) which refers to a homo- or copolymer of methyl methacrylate (MMA) that comprises at least 50%, preferably at least 60%, more preferably at least 70%, advantageously at least 80% and more advantageously at least 90% by weight of methyl methacrylate.

In the case of a PMMA (polymer) and MMA (monomer), the boiling temperature of the MMA is approx. 100°C and the boiling temperature of the dimers and the trimers are approx. 200°C and 300 °C respectively.

Hence, in such case, the depolymerization method is implemented such that the temperature of the gas stream in the downstream section of the separator 14 is comprised between 100°C and 280°C, in particular between 140 and 180 °C.

During implementation of the depolymerization method, the absolute pressure of the gas flow inside the separator 14 is preferably comprised between 0.35 bar and 5 bar, preferably between 0.5 bar and 4 bar, still preferably between 1 bar and 3 bar.

Advantageously, the depolymerization method comprises spraying into the separator 14 liquid monomer that has been retrieved from the condenser 16, the liquid monomer being sprayed into the gas stream, and preferably facing the gas stream.

Optionally, the depolymerization method comprises spraying a fraction of the liquid monomer recovered by the condenser 16 into the separator 14.

In such case, the depolymerization system 10 is configured for spraying a fraction of the liquid monomer recovered by the condenser 16 into the separator 14.

The depolymerization system 10 comprises for example a monomer return line 18 configured for collecting liquid monomer provided by the condenser 16 and spraying the liquid monomer inside the separator 14.

Optionally, the depolymerization method comprises a step of reinjecting liquid monomer collected at the liquid outlet 16B of the condenser 16, into gas entering the inlet 16A of the condenser 16, from the outlet of the condenser 16 to the inlet of the condenser 16.

This allows reducing the temperature of the gas entering the condenser 16 and increasing the efficiency of the condenser 16.

In such case, the depolymerization system 10 comprises a reinjection line 19 configured for collecting liquid monomer at the liquid outlet 16B of the condenser 16 and reinjecting this liquid monomer into the gas inlet 16A of the condenser 16.

Optionally, the depolymerization method comprises a step of purifying the condensate provided by the condenser 16 to remove solid particles from the condensate provided by the condenser 16.

In such case, the depolymerization system 10 comprises a purifying device 20 configured for removing solid particles from the condensate provided by the condenser 16.

The liquid monomer sprayed into the separator 14 may be purified liquid monomer, purified to remove solid particles before spraying into the separator 14, or raw liquid monomer directly sprayed into the separator as provided by the condenser 16.

Correspondingly, in the depolymerization system 10, the monomer return line 18 is fed with purified condensate provided by the purifying device 20, as illustrated on Figure 1, or alternatively the monomer return line 18 is fed with raw condensate provided directly by the condenser 16.

Preferably, the liquid monomer is sprayed into the separator 14 such that the ratio of the mass rate of liquid monomer sprayed into the separator 14 to the mass rate of gas stream is equal or inferior to 93%, still preferably equal or inferior to 83%, still preferably equal of inferior to 73%, still preferably equal or inferior to 63%, still preferably equal or inferior to 53%.

Preferably, the liquid monomer is sprayed into the separator 14 such that the mass rate of liquid monomer sprayed into the separator 14 to the mass rate of gas stream is comprised equal or superior to 10%, still preferably equal or superior to 20%, preferably equal or superior to 30%, still preferably equal or superior to 40%, still preferably equal or superior to 50%.

Optionally, as an alternative or a complement to the spraying of liquid monomer, the depolymerization method comprises spraying liquid water inside the separator 14. The benefit is similar to that of spraying liquid monomer inside the separator 14.

When the case may be, the liquid monomer reinjected into the gas inlet 16A of the condenser may be purified liquid monomer, purified to remove solid particles before reinjection into the condenser 16, or directly as provided by the condenser 16.

Correspondingly, in the depolymerization system 10, the reinjection line 19 is fed with purified condensate provided by the purifying device 20, as illustrated on Figure 1, or alternatively the reinjection line 19 is fed with raw condensate provided directly by the condenser 16.

The pyrolysis reactor 12 is configured for processing the feed material with a maximum mass flow rate and thus for providing the gas stream with a maximum mass flow rate.

Further, in any section of the internal flow passage of the separator 14, the gas stream flowing in the separator 14 has a linear velocity that is determined as the volume of gas produced by the pyrolysis reactor 12 by time unit divided by the area of the cross-section of said section of the internal flow passage of the separator 14.

Preferably, the depolymerization method is implemented such that the linear velocity of the gas stream in the separator 14 is lower than a maximum linear velocity at least in the section of the internal flow passage of the separator 14 having the largest cross-section.

In a particular embodiment, the section of the internal flow passage of the separator 14 having the largest cross-section is a downstream section of the internal flow passage of the separator 14.

In one embodiment, in particular if the polymer is PMMA, the maximum linear velocity is of 0.50 m/s, preferably 0.15 m/s, in particular of 0.10 m/s.

The linear velocity of the gas stream in any section of the internal flow passage of the separator 14 is a function of the mass flow rate of the gas stream or the volume flow rate of the gas stream and of the area of the cross-section of said section of the internal flow passage of the separator 14. The mass flow rate is related to the volume flow rate and the mass density of the gas stream at the operating pressure.

The volume flow rate of the gas stream is a function of the feed rate of the pyrolysis reactor 12, the depolymerization temperature of the polymer and the molar weight of the polymer.

Advantageously, the area of the largest cross-section of the internal flow passage of the separator 14 is for example chosen as a function of the maximum feed rate of the pyrolysis reactor 12, the depolymerization temperature of the polymer and the molar weight of the polymer such that the linear velocity of the gas stream in the largest cross-section of the internal flow passage of the separator 14 is lower than the maximum linear velocity at the maximum feed rate of the pyrolysis reactor.

Preferably, the largest cross-section of the internal flow passage of the separator 14 has an area that is at least 0.001 times the maximum mass flow rate of the gas stream provided by the pyrolysis reactor 12, the area being expressed in square meters (m²) and the maximum mass flow rate being expressed in kilograms per hour (kg/h).

Preferably, the area of the largest cross-section of the internal flow passage of the separator 14 is at least ten times the area of the smallest cross-section in the separator 14.

These parameters allow limiting the linear velocity of the gas stream in the separator 14, thus favoring deposition of dust and droplets carried by the gas stream and thus separation of these droplets from the gas stream. Indeed, the lower the linear velocity of the gas stream, the less easily droplets and dusts are carried by the gas stream.

Advantageously, the depolymerization method comprises slowing down the gas stream progressively inside the separator 14.

This is obtained e.g. by progressively increasing a cross-section of the internal flow passage of the separator 14 from the separator gas inlet 14A to the separator gas outlet 14B.

In such case, the downstream section of the internal flow passage of the separator 14 is the section of the internal flow passage of the separator 14 having the largest cross-section.

Advantageously, the residence time of the gas stream in the separator 14 is comprised between 1 s and 30 s, preferably between 5 s and 25 s, still preferably between 10 s and 20 s.

The residence time is the time that is necessary for gas stream to flow from the separator inlet 14A to the separator outlet 14B.

The residence time is a function of the length of the internal flow passage of the separator 14, the average temperature, the mass flowrate or the volume flowrate and the molecular weight, and so of the volume of the separator 14.

The gas stream generated by the pyrolysis reactor 12 may contain a mist formed of mist droplets. Mist droplets may form around dust particles present in the gas stream. Dust particle may be due to impurities in the feed material containing the polymer that is intended to be depolymerized. Mist droplets present in the gas steam may comprise liquid monomer, liquid dimer or liquid trimer carried onto dust particles present in the gas stream.

Advantageously, the depolymerization method comprises demisting the gas stream (e.g. with passing the gas stream through a demister or demisting device) and/or desublimating the gas stream (i.e. with passing the gas stream through a desublimator or desublimation device).

Demisting is different from condensing monomer in gaseous state to monomer in liquid state. Demisting consists in collecting droplets in liquid state that are already present in the gas stream, like droplets in a fog.

Demisting is obtained for example by favoring coalescence of mist droplets present in the gas stream, e.g. by defining small passages forcing the droplets to flow closer to each other and then to coalesce.

Demisting is performed e.g. by providing at least one demisting device, each demisting device being configured for favoring coalescence of the mist droplets such that the mist droplets become too heavy for being transported by the gas stream.

Each demisting device may comprise a cyclone effect chamber, at least one grid, at least one mesh, at least one piece of foam, at least one porous monolith and/or at least one baffle for capturing mist droplets, e.g. by favoring coalescence of the mist droplets.

Demisting may be performed e.g. inside the separator 14, between the separator 14 and the condenser 16 or downstream the condenser 16.

Optionally, the depolymerization method comprises demisting the gas stream inside the separator 14.

In such case, the separator 14 comprises an internal demisting device located inside the separator 14.

Optionally, the depolymerization method comprises demisting the gas stream downstream the separator 14 and upstream the condenser 16, i.e. between the separator 14 and the condenser 16.

In such case, the depolymerization system 10 comprises an upstream demisting device 22 arranged serially between the separator 14 and the condenser 16, the upstream demisting device 22 being configured for capturing mist droplets present in the gas stream.

The upstream demisting device 22 is arranged downstream the pyrolysis reactor 12 and upstream the separator 14.

Optionally, the depolymerization method comprises demisting the gas stream downstream the condenser 16.

In such case, the depolymerization system 10 comprises a downstream demisting device 24 arranged downstream the condenser 16, the downstream demisting device 24 being configured for removing droplets present in the gas stream.

Desublimation differs from demisting as it provides retaining a gaseous part of a gas stream as solids.

Desublimation comprises passing the gas stream through a chamber containing lamellas or fins which are cooled such that the product to be separated from the rest of the gas stream desublimates around or below its triple point and adheres to lamellas or fins in crystalline form.

The desublimator progressively charges with the separated product in crystalline form and can drained by heating so that the crystallized separated product melts. It requires that the separated product to be trapped can be heated to melt without polymerizing.

In the case of depolymerization of PMMA, providing a desublimator could be appropriate to remove Terephthalic acid of DimethylTerephthalate that could be generated by PET contamination and present in the gas stream.

Providing a desublimator is appropriate, for example in case there is a risk of contamination with polyolefins, like PE, which can generate waxes that have a high melting point and that can be trapped in the condenser 16.

A desublimation unit may comprises two desublimators fluidly connected in parallel, such that one of the two desublimators can be maintained in operation while the other is drained, thus allowing continuous operation of the depolymerization system 10.

Optionally, the depolymerization method comprises passing the gas stream through a desublimator to trap contaminants present in the gas stream.

In case a desublimator is provided, the latter is preferably installed between the separator 14 and the condenser 16.

As illustrated on Figure 1, the depolymerization system 10 comprises at least one desublimator 25, each desublimator 25 provided between the separator 14 and the condenser 16 to process the gas stream flowing from the separator 14 to the condenser 16.

The depolymerization system 10 comprises for example two desublimators 25 provided in parallel with each other between the separator 14 and the condenser 16 such the each desublimator 25 can process the gas stream, when the other is being discharged.

Optionally, the depolymerization method comprises evacuating non-condensated product provided by the condenser 16.

In such case, the depolymerization system 10 comprises an evacuation device 26 for evacuating the non-condensate product. The evacuation device 26 is arranged to receive the gas stream provided by the condenser 16 at the gas outlet 16B of the condenser 16.

If a downstream demisting device 24 is provided, the downstream demisting device 24 is preferably arranged serially between the condenser 16 and the evacuation device 26.

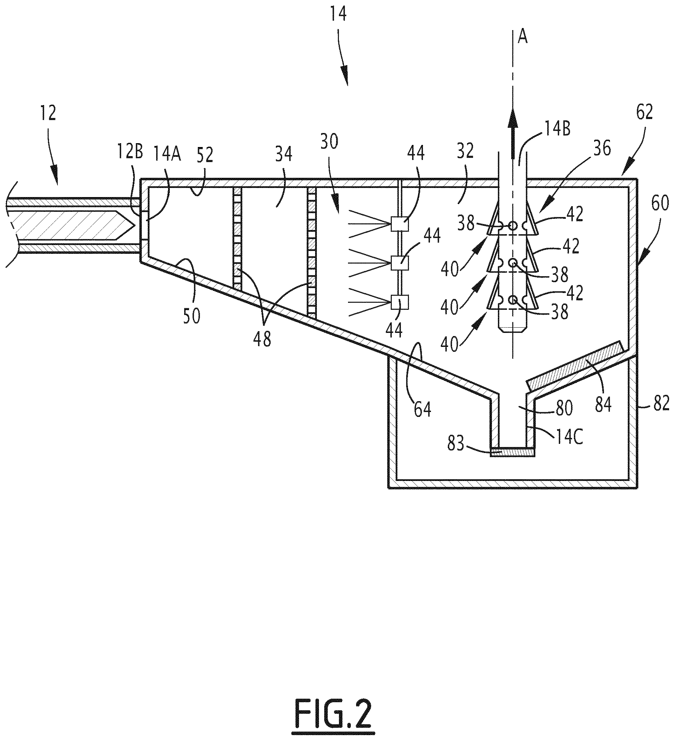

As separator 14 suitable for use in the depolymerization system 10 for implementing the depolymerization method is illustrated on Figure 2.

The separator 14 comprises the internal flow passage 30 configured for circulation of the gas stream between the separator inlet 14A and the separator outlet 14B.

In one example, as illustrated on Figure 2, the pyrolysis reactor outlet 12B opens directly into the separator inlet 14A such that the gas stream generated by the pyrolysis reactor 12 flow directly from the pyrolysis reactor outlet 12B.

Preferably, when the pyrolysis reactor 12 has at least one extruding screw, the extruding screw is inserted or protrudes into the separator inlet 14A in order to avoid blockage of the reactor outlet 12B by impurities.

In other words, the pyrolysis reactor outlet 12B is directly fluidly connected to the separator inlet 14A, such that the gas stream generated by the pyrolysis reactor 12 flows directly from the pyrolysis reactor outlet 12B connected directly to the separator inlet 14A without passing by any other fluid conducting element.

The separator 14 comprises, for example, a separation chamber 32 extending around a central axis A, the separator 14 being configured such that the gas stream flows in the separation chamber 32 circularly around the central axis A.

The separator 14 is thus configured for implementing a cyclone effect in the separation chamber 32.

With such a configuration, the gas stream flows faster at the periphery of the separation chamber 32 and slower at the center of the separation chamber 32 whereby impurities present in the gas stream tend to stay at the periphery of the separation chamber 32.

The separator 14 comprises for example an inlet duct 34, configured for receiving the gas stream generated by the pyrolysis reactor 12. The inlet duct 34 extends from the separator inlet 14A that defines the upstream end of the inlet duct 34. The inlet duct 34 forms the upstream section of the internal volume 30 of the separator 14.

In view of promoting the circular flow of the gas stream in the separation chamber 32, the inlet duct 34 emerges for example into the separation chamber 32 such as to impart to the gas stream in the separation chamber 32 a rotational movement around the central axis A.

In a particular embodiment, the inlet duct 34 opens into the separation chamber 32 such that the gas stream emerges in the separation chamber 32 along an inlet direction that defines a non-zero angle with a radial direction that is radial relative to the central axis A.

In a variant in which the separator 14 has no inlet duct 34, the separator inlet 14A opens for example into the separation chamber 32 such as to impart to the gas stream in the separation chamber 32 a rotational movement around the central axis A.

In a particular embodiment, the separator inlet 14A opens for example into the separation chamber 32 such that the gas stream emerges in the separation chamber 32 along an inlet direction that defines a non-zero angle with a radial direction that is radial relative to the central axis A.

Advantageously, the inlet duct 34 extends around the central axis A. This allows obtaining a cyclone or rotating effect in the inlet duct 34

The separator 14 is preferably configured for collecting the gas stream at the center of the separation chamber 32 and for providing the gas stream collected at the center of the separation chamber 32 to the separator outlet 14B.

The separator 14 comprises, for example, a collecting tube 36 located at the center of the separation chamber 32, the collecting tube 36 extending along the central axis A and having at least one inlet opening 38 for the gas stream present in the separation chamber 32 to enter the collecting tube 36, the collecting tube 36 being fluidly connected to the separator outlet 14B for providing the collected gas stream to the separator outlet 14B.

The collecting tube 36 is for example provided with at least one inlet section 40, each inlet section 40 comprising one inlet opening 38 or several inlet openings 38 distributed circumferentially around the inlet section 40 of the collecting tube 36.

The collecting tube 36 is for example provided with a plurality of inlet sections 40 distributed along the collecting tube 36, each inlet section 40 comprising one inlet opening 38 or several inlet openings 38 distributed circumferentially around the inlet section 40 of the collecting tube 36.

In a preferred embodiment, the collecting tube 36 is provided with at least one frustoconical baffle 42, each frustoconical baffle 42 having an upper edge of smaller diameter connected to the collecting tube 36 and a lower edge of larger diameter that is free, the baffle 42 extending around a respective inlet section 40.

Preferably, each inlet section 40 is associated with a respective frustoconical baffle 42 extending around this inlet section 40.

Preferably, the upper edge of each frustoconical baffle 42 is located above each inlet opening 38 of the corresponding inlet section 40, the lower edge of said frustoconical baffle 42 being located below each inlet opening 38 of the corresponding inlet section 40

Each frustoconical baffle 42 forces the gas stream to flow below the lower edge of the frustoconical baffle 42 before entering the inlet opening(s) 38 of the corresponding inlet section 40.

Each frustoconical baffle 42 favors impurities to fall at the bottom of the separation chamber 32 and/or to deposit onto the frustoconical baffle 42.

Preferably, each inlet opening 38 has an area comprised between 3 and 79 mm², and preferably between 12 and 51 mm²

Preferably, each inlet opening 38 has a circular contour with a diameter comprises between 2 and 10 mm, preferably between 4 and 8 mm.

Preferably, the cumulated area of the inlet openings 38 (i.e. the sum or the area of all the inlet opening 38) is comprises between 0.01 and 0.1 times the area of the cross-section of the upstream section of the internal flow passage 30 of the separator 14.

In the separator 14 of Figure 2, the upstream section of the internal flow passage 30 of the separator 14 corresponds to the upstream end of the inlet duct 34.

Advantageously, the separator 14 is configured such that largest cross-section of the separator 14 is located in the downstream section of separator 14.

In the separator 14 as illustrated on Figure 2, the downstream section the separator 14 is defined by the separation chamber 32.

In one example in which the separator 14 comprises the inlet duct 34 and the separation chamber 32, the inlet duct 34 has a cross-section that increases progressively from the upstream end of the inlet duct 34 receiving the gas stream to the downstream end of the inlet duct 34 opening into the separation chamber 32.

The gas stream is thus slowed-down progressively in the inlet duct 34 until the gas stream reaches the separation chamber 32.

The inlet duct 34 has for example a height that increases progressively in the downstream direction and/or a width that increases progressively along the inlet duct 34 in the downstream direction such as to increase the area of the cross-section of the inlet duct 34 in the downstream direction.

Advantageously, as illustrated on Figure 2, the separator 14 is configured for spraying liquid monomer in the gas stream inside separator 14. The separator 14 is configured for spraying one or several spray jets in the gas stream inside the separator 14.

To this end, the separator 14 comprises at least one spraying nozzle 44 for spraying liquid monomer inside the separator 14. Each spraying nozzle 44 is configured for spraying a spray jet.

The direction of each spray jet can be parallel or perpendicular or oblique to the gas flow. In one example, each spray jet is facing the gas flow, i.e. sprayed towards the upstream of the gas stream.

Each spraying nozzle 44 is fluidly connected to the condenser 16, more specifically to the return line 18.

Each spraying nozzle 44 is arranged for example to spray the liquid monomer in the separation chamber 32.

As illustrated on Figure 2, the separator 14 comprises for example spraying nozzles 44 arranged to spray the liquid monomer at the entry of the separation chamber 32, i.e. where the inlet duct 34 connects to the separation chamber 32, here in the upstream direction of the gas stream.

Advantageously, the separator 14 integrates an internal demisting device 46 located inside the separator 14 for deposition of mist droplets transported by the gas stream.

The internal demisting device 46 comprises for example at least one grid 48 extending across a cross-section of the separator 14 such that the gas stream flows through the openings of the grid 48.

In one example in which the separator 14 comprises the inlet duct 34, each grid 48 is for example located inside the inlet duct 34.

As illustrated on Figure 2, the separator 14 comprises two grids 48 arranged in the inlet duct 34. The two grids 48 are located at a distance from each other along the inlet duct 34.

The inlet duct 34 is delimited between a lower surface 50 and an upper surface 52.

In one example, the duct lower surface 50 is inclined and/or the duct upper surface 52 is inclined.

In particular, the duct lower surface 50 is inclined and/or the duct upper surface 52 is inclined such that the cross-section of the inlet duct 34 increases from the upstream end of the inlet duct 34 towards the downstream end of the inlet duct 34.

As illustrated on Figure 2, the duct lower surface 50 is inclined such that it descends from the upstream end of the inlet duct 34 towards the downstream end of the inlet duct 34. Mist droplets depositing on the duct lower surface 50 will tend to flow towards the separation chamber by gravity.

In one example, the inlet duct 34 extends around the separation chamber 32, on at least a portion of the circumference of the separation chamber 32.

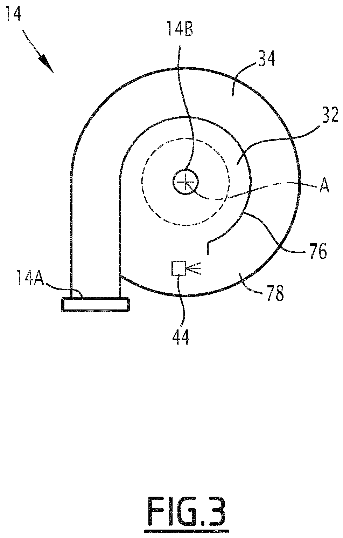

As illustrated on Figure 3, the inlet duct 34 extends around the separation chamber 32 on approximately 270° around the central axis A.

In operation, the gas stream is hot when entering the separator 14 and the temperature of the gas stream tends to lower when travelling through the separator 14 due to thermal loss, even if the separator 14 is properly thermally insulated. If spraying of liquid monomer into the separator 14 is provided, this spraying tends to further cool the gas stream in the separator 14 due to vaporization of the liquid monomer that is sprayed into the hot gas flow.

The inlet duct 34 partially surrounding the separation chamber 32 is beneficial for maintaining the gas stream sufficiently hot in the separator 14 in particular in the separation chamber 32.

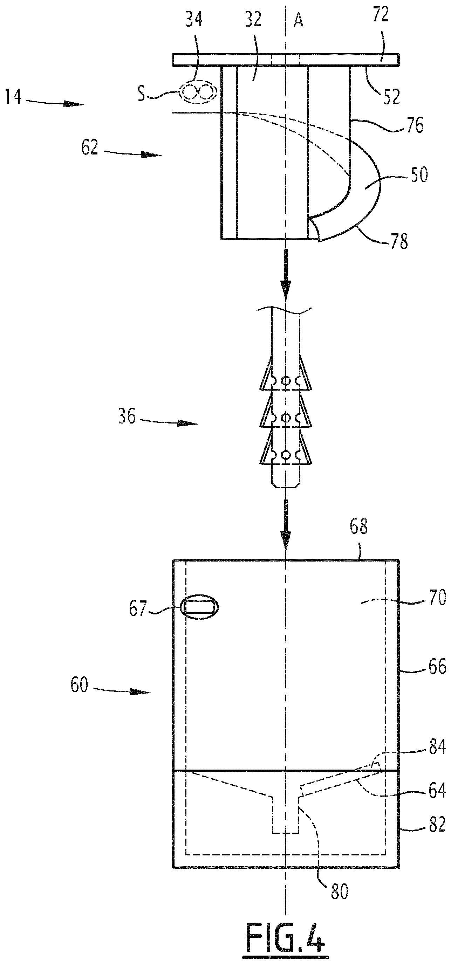

As illustrated on Figure 4, in one exemplary embodiment, the separator 14 comprises a bottom part 60 and a top part 62 to be fitted to the bottom part 60 for forming the separator 14.

The bottom part 60 comprises a bottom 64 and a peripheral wall 66 extending upwardly from the periphery of the bottom 64, the peripheral wall 66 defining an upper opening 68 and an internal volume 70. The peripheral wall 66 is provided with a side opening defining the inlet opening 14A of the separator 14. The peripheral wall 66 is for example cylindrical and of circular cross-section.

The top part 62 comprises a lid 72 for closing the upper opening 68 and an internal structure 74 extending downwardly from the lid 72 for fitting inside the bottom part 60 upon closing the upper opening 68 with the lid 72. The internal structure 74 is configured to define the separation chamber 32 and the inlet duct 34 inside the internal volume 70 of the bottom part 60.

The internal structure 74 comprises a partition wall 76 extending downwardly from the lid 72 along the central axis A for internally delimiting the separation chamber 32 and externally delimiting the inlet duct 34 separated by the peripheral wall 66, and a duct element 78 extending around the partition wall 76 for delimiting the lower surface 50 of the inlet duct 34, the upper surface 52 of the inlet duct 34 being delimited by a peripheral region of the lower face of the lid 72.

The partition wall 76 has an inner face 74A defining a lateral wall of the separation chamber 34 and an outer face 76B defining a lateral wall of the inlet duct 34.

In view along the central axis A, the partition wall 76 extends around the central axis A, approximately on 270° on Figure 3.

The inlet duct 34 is delimited between the partition wall 76, the peripheral wall 66, the duct element 78 and the lid 72.

The peripheral wall 66 is provided with a connector 67 for connection to the outlet of the pyrolysis reactor 12. The contour of the connector 67 is represented in dotted line as S to show its position relative to the upstream end of the inlet duct 34.

The lid 72 is perpendicular to the central axis A and the duct element 78 extends around the central axis A with a top surface of the duct element 78 being inclined relative to the central axis A such that the height of the inlet duct 34 increases progressively from the upstream end of the inlet duct 34 to the outlet end of the inlet duct 34, and the cross-section of the inlet duct 34 increases progressively from the upstream end of the inlet duct 34 to the outlet end of the inlet duct 34.

The duct element 78 extends for example helically around the central axis A of the separation chamber 32.

In view along the central axis A, the partition wall 76 extends for example along a portion of a circle or along a portion of a spiral with getting closer to the central axis A in the downstream direction of the inlet duct 34 delimited by the partition wall 76.

The circular extension of the partition wall 76 is used e.g. for defining an inlet duct 34 of constant width in the downstream direction of the inlet duct 34 and the spiral extension of the partition wall 76 is used e.g. for defining an inlet duct 34 of progressive increasing width in the downstream direction of the inlet duct 34.

The bottom 64 of the bottom part 60 defines the lower surface of the separation chamber 32.

The bottom 64 is provided with an evacuation opening 80 for evacuation of the impurities separated from the gas stream in the separator.

Preferably, the bottom 64 is shaped for the impurities to fall by gravity towards the evacuation opening 80. As illustrated on Figure 4, the bottom 64 is frustoconical with tapering downwardly to the evacuation opening 80.

Optionally, the separator 14 comprises a collection pot 82 that is configured for being fitted under the bottom part 60 for collecting impurities falling into the evacuation opening 80.

Preferably, the collection pot 82 is removably connected to the bottom 64. Hence, the collection pot 82 can be removed for emptying the collection pot 82.

Optionally, the evacuation opening 80 is equipped with a valve 83, for example a slide valve, arranged for selectively closing or opening the evacuation opening 80. This allows disconnecting the collection pot 82 after closing the valve 83 while the separator 14 is still in operation.

The collection pot 82 may be equipped with a gas purging line and/or the collection pot 82 may be actively cooled to allow safely removing it and replacing it.

In one example, the separator 14 is provided with a scrapping device configured for scrapping products deposited in the separator 14 to an evacuation opening. The scrapping device is configured for example for scrapping a bottom of the separator 14.

As illustrated on Figures 2 and 4, a scrapping device 84 is for example configured for scrapping products depositing on the bottom wall 64 of the bottom part 60 towards the evacuation opening 80.

The separator 14 is not limited to the example illustrated on Figures 2 - 4, other variants or options being possible.

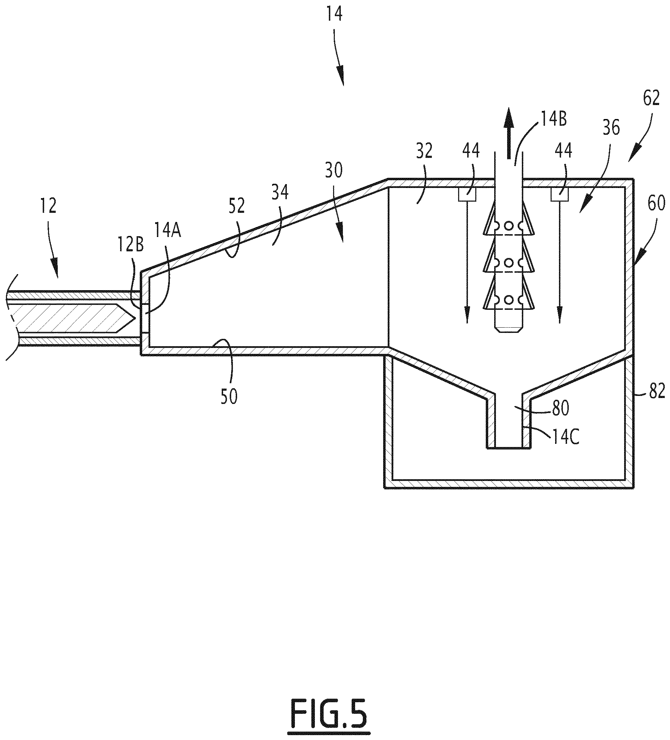

The separator 14 of Figure 5, on which numeral references to similar elements are the same, differs from that of Figure 2 - 4 namely in that the inlet duct 34 is delimited by a lower surface 50 that extends in a plane perpendicular to the central axis A and an upper surface 52 that is inclined to ascend from the upperstream end of the inlet duct 34 to the downstream end of the inlet duct 34.

The separator 14 of Figure 5 differs from that of Figure 2 - 4 in that the spraying device 42 is arranged to spray the liquid monomer at a location proximate to the central axis A, in particular around the central axis A. The spraying device 42 comprises for example a plurality of spraying nozzles 44 distributed around the central axis A.

If a collection tube 36 is provided as illustrated on Figure 5, the spraying device 42 is for example configured for spraying the liquid monomer around the collection tube 36.

Spraying of liquid monomer retrieved from the condenser 16 into the separator 14 may be useful for preventing clogging of one or several demisting grids 48 provided inside the separator 14 over time, in particular by spraying the liquid monomer onto the desmisting grids 48.

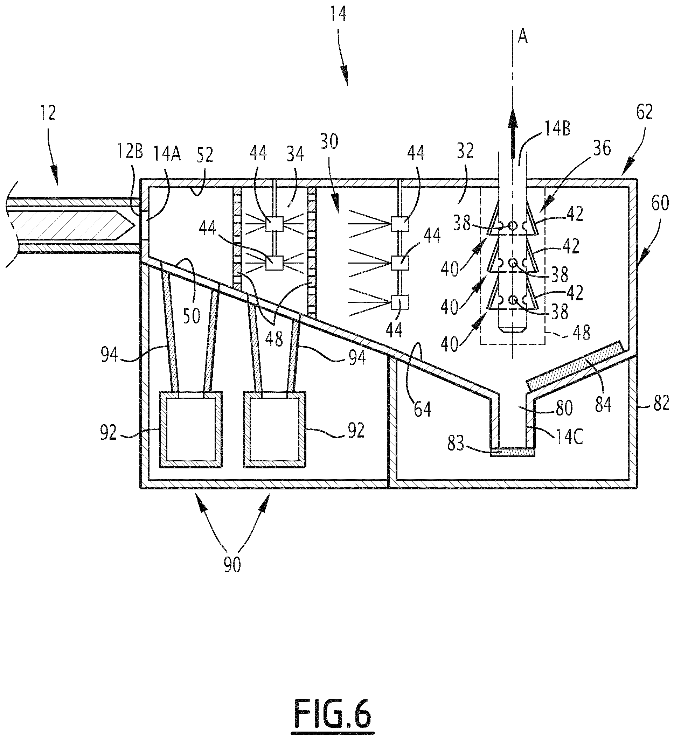

The separator 14 of Figure 6, on which numeral references to similar elements are the same, comprises spraying nozzles 44 orientated to spray liquid monomer retrieved from the condenser 16 onto the most downstream demisting grid 48 that is provided in the inlet duct 34 of the separator 14.

Optionally, as illustrated on Figure 6, the separator 14 comprises one or several spraying nozzles 44 arranged for spraying liquid monomer retrieved from the condenser 16 on a face of the most upstream demisting grid 48 that is provided in the inlet duct 34 of the separator 14.

Optionally, as illustrated on Figure 6, the separator 14 comprises one or several spraying nozzles 44 arranged for spraying liquid monomer retrieved from the condenser 16 in an intermediate space delimited between two demisting grids 48 that are provided in the inlet duct 34 of the separator 14, preferably with spraying liquid monomer onto one or both of these two demisting grids 48.

Optionally, as illustrated on Figure 6, the separator 14 comprises a demisting grid 48 provided in the downstream section of the separator, in particular a demisting grid 48 that surrounds the collecting tube 36.

This demisting grid 48 is, for example, arranged in a tubular shape and fitted around the collecting tube 36 such that the gas stream has to pass through this demisting grid to attain the collecting tube 36.

The gas stream entering the separator 14 may contain solid particles due to impurities of the raw material that is being depolymerized in the pyrolysis reactor 12.

Optionally, the separator 14 is provided with at least one solid particles collector 90 that is preferably arranged in the inlet duct 34 of the separator 14, preferably upstream a demisting grid 48 provided in the inlet duct 34 of the separator 14.

Each solid particles collector 90 comprises e.g. a collecting pot 92 connected to the separator 14 by a collecting duct 94 such that solid particles fall by gravity into the collecting pot 92. The collecting duct 94 connects for example to a lower wall 50 of the inlet duct 34 of the separator 14.

The collecting pot 92 is preferably removably connected to the separator 14. This allows emptying the collecting pot 92 when solid particles are collected therein or replacing the collecting pot 92 by a new one.

Preferably, each solid particles collector 90 is located in a climatic chamber 96 that is controlled to maintain therein a temperature equal to or above the boiling temperature of the monomer.

The separator 14 may be provided with one or several solid particles collectors 90.

As illustrated on Figure 6, the separator 14 is provided with two solid particles collectors 90 each connected to the inlet duct 34 of the separator 14 at a respective location along the inlet duct 34 of the separator 14.

When a plurality of solid particles collectors 90 are provided, each solid particles collector may be connected to the inlet duct 34, of the separator 14, upstream a respective one of a plurality of demisting grids 48 provided in the inlet duct 34 of the separator 14.

As illustrated on Figure 6, the separator 14 is provided with two demisting grids 48 extending across the inlet duct 34 of the separator 14 and with two solid particles collectors 90 each connected to the inlet duct 34 of the separator 14 upstream a respective one of these two demisting grids 48.

One of the solid particles collectors 90 is connected to the upstream section 34 of the separator 14 upstream the most upstream demisting grid 48 and the solid particles collectors 90 is connected to the inlet duct 34 of the separator 14 between two demisting grids 48.

When a plurality of solid particles collectors 90 are provided, they can be housed in the same climatic chamber 96.

As illustrated on Figure 6, the separator 14 is provided with two solid particles collectors 90 arranged in the same climatic chamber 96.

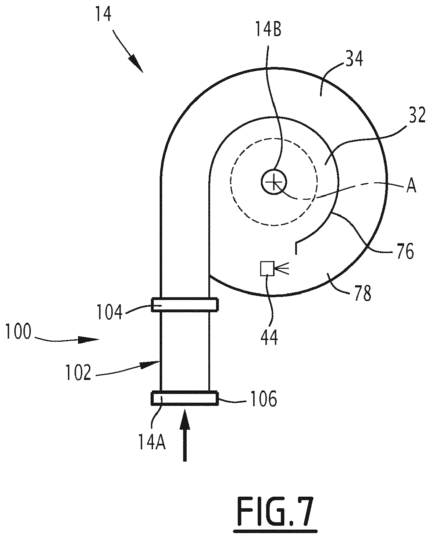

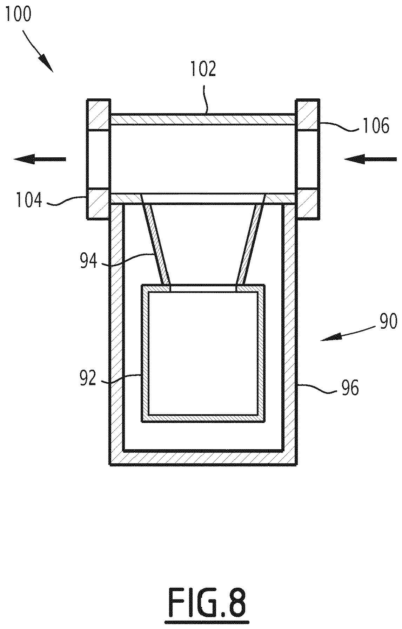

As illustrated on Figure 7 and 8, advantageously, the separator 14 is configured with a removable inlet module 100 that comprises an inlet tube 102 defining the inlet portion of the inlet duct 34 to be connected to the outlet 12A of the pyrolysis reactor 12 and a solid particle collector 90 connected to the inlet tube 102. The inlet module 100 preferably comprises a climatic chamber 96 attached to the inlet tube 10.

One end of the inlet tube 102 is for example provided with a first flange 104 for removable connection to the remainder of the separator 14 and with a second flange 106 for removable connection to the outlet 12A of the pyrolysis reactor 12.

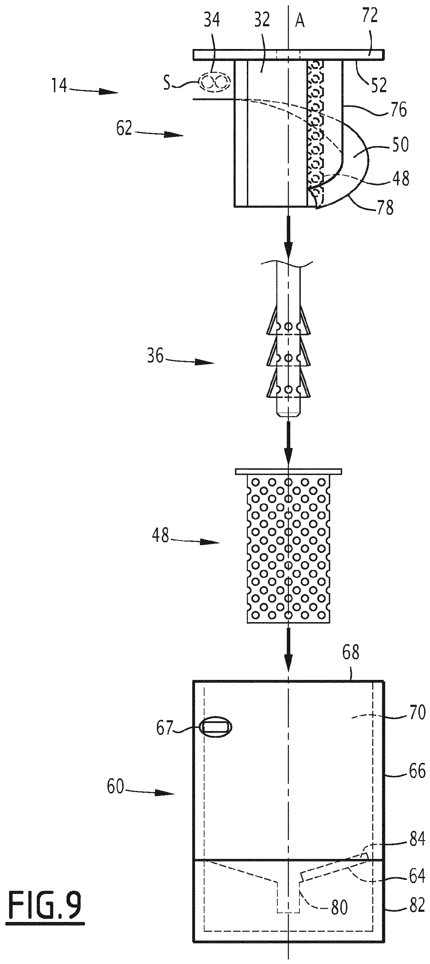

As illustrated on Figure 9, the separator 14 may comprise a demisting grid 48 extending across the internal flow passage 30 at the junction between the inlet duct 34 and the chamber 32.

The demisting grid is for example provided on the top part 62 with being attached on the partition wall 76 and the duct element 78.

Preferably, the demisting grid 48 is arcuate such that the demisting grid is bulging in the upstream direction. The demisting grid 48 as for example two rectilinear vertical side edges and a top edge and a bottom edge that are arcuate. This provide a larger free cross-section for the passage of the gas through the demisting grid 48.

As illustrated on Figure 9, a tubular demisting grid 48 is also provided inside the chamber 32 and around the collecting tube 36 to cover the collecting tube 36.

Examples have been implemented in a depolymerization unit comprising a pyrolysis reactor 12, and separator 14 and a condenser 16.

The pyrolysis reactor 12 is provided as a twin-screw extruder with co-rotation of the screws and intermeshing of the screw.

The twin-screw extruder includes the screw extending through a barrel, an electric heater providing the heating energy, which is installed around the barrel, and thermocouples for temperature control which are also installed in the barrels. The barrel is for example formed by barrel segments.

The twin-screw extruder, and in particular its heaters, are configured for generating a temperature profile along the screws, with a maximum profile temperature at an intermediate section along the screws.

The twin-screw extruder is a TEX44 having a 47mm screw diameter. For industrial production line, a TEX90 having a 96.5mm screw diameter may be used according to the requested feeding rate. TEX extruders are marketed by The Japan Steel Works, Ltd which also offers larger units.

The material to be depolymerized is fed into the twin-screw extruder at a hopper barrel by gravimetric screw feeders. The material provided to the gravimetric screw feeders is previously crushed to a size of about 5 to 10 mm.

The twin-screw extruder has along its length a melting section followed by a depolymerization section.

In operation, the material is molten in the melting zone of the twin-screw extruded by shear stress from screw and heat from the heaters, then the melted material is transported to the depolymerization section.

The depolymerization section of the pyrolysis reactor 12 can achieve a temperature of 550°C.

At the depolymerization section, the polymer is depolymerized by heater energy assisted by the shear stress from screw (mechanical energy), and transformed into the MMA monomer gas which is moved to downstream equipment.

PMMA mixtures in the form of crushed particles were fed through the gravimetric feeders. Two to four independent feeders were loaded with cast, injection and/or extrusion PMMA.

The following operating conditions were used during comparative examples and the examples of the invention : a feed rate of the material in the pyrolysis reactor 12 comprised between 50 and 100 kg/h, a rotation speed of the screws of the pyrolysis reactor 12 of about 800 rotations per minute, and a maximum profile temperature in the pyrolysis reactor 12 of 490 °C or less.

In the comparative examples detailed below, the separator 14 is a dead-end and the gases generated during the depolymerization are withdrawn through an exit pipe which is located between the outlet of the pyrolysis reactor 12 and the separator 14.

The separator 14 is in the shape of a tank having cylindrical lateral wall and a hemispheric bottom. The extruding screws of the pyrolysis reactor 12 are oriented radially with respect to the cylindrical lateral wall. The extruding screws of the pyrolysis reactor 12 were inserted into the separator inlet 14A in order to avoid the blockage of the reactor outlet 12B by impurities.The cylindrical tank has a 817 mm internal diameter and 665 mm internal height. The inner volume of the separator 14 is 300 liters.

Temperature of the gases inside the separator 14 are measured using a thermocouple.

In examples according to the invention detailed below, the pyrolysis reactor 12 and the condenser 16 are is the same, but the separator 14 differs from that of the comparative example in that the pyrolysis reactor 12 is connected to the separator 14 such that the screws are orientated tangentially with respect the central axis A of the lateral wall 66 as in the example illustrated on Figure 3.

In addition, as illustrated on Figure 3, the separator 14 is provided as a tank equipped internally with a separation wall 76 defining inside the tank a chamber 32 and an inlet duct 34 from an inlet of the separator 14 to the chamber 32 with extending around the chamber 32, the separation wall 76 extending along a cylindrical surface having a radius of 300 mm, a collection tube 36 as illustrated on Figure 2 being provided centrally in the chamber 32. The area of the cross-section of the inlet duct 34 varies from the inlet of the separator 14 to the chamber 32 from about 344 cm² to about 470 cm².

A first thermocouple is provided for measuring the temperature of the gas flow half way of the inlet duct 34 and a second thermocouple is provided close to the bottom of chamber 32 where liquid accumulates, to measure the temperature of the liquid, or, in the absence of liquid, the temperature of the gas flow at the end of the inlet duct 34.

### Comparative example 1

A colored mix of cast and extrusion PMMA sheets, in a 80/20 mass ratio was fed to the depolymerization unit. The depolymerization unit was first operated at 470 °C, at a feed rate of 60 kg/h, for about 1 hour. Then the temperature was increased to 490 °C and the depolymerization unit was operated in this condition for 1 hours and 30 minutes. The depolymerization was shut down, and restarted after 2 h and operated at 490 °C for another 1 hour and 30 minutes.

The efficiency of the condenser 16 decreased over time, as detected by an increase of the temperature in the exit line. The temperature of the condensed MMA was also increasing and reached the maximum preset limit, at which time the pilot plant was shutdown.

The condenser 16 was deconstructed for inspection. A distributor plate above condenser tubes was completely covered with a black dust. Seven out of the twenty four tubes of the condenser 16 were completely plugged. The tubes were cleaned with a solvent for the following test.

During this test, the temperature in the separator 14 varied between 30 and 50.7 °C. The temperature at the entrance of the condenser initially increased and stabilized between 365 and 393 °C.

The Methyl Methacrylate and Methanol contents were measured by Gas Chromatography using an internal standard and a calibration. They were found at 93 +/-2 wt %, and 0.4 wt % respectively.

### Example 2 according to the invention

The depolymerization unit was fed with a cast material containing PMMA and was used for about 2 hours at a feed rate of 70 and 90 kg/h. Without cleaning the depolymerization unit in between, the depolymerization unit was re-started at 500 °C and 1.5 bars, and first with a feed rate of 80 kg/h for about 30 minutes then increased to 90 kg/h for about 1 hour and then further increased it to 100 kg/h for the rest of the test. The depolymerization unit operated at a pressure of 1.5 bars for about 30 minutes in these conditions, then the pressure was reduced to 1.0 bar for 30 minutes and further reduced to 0.5 bar and kept in these conditions for also 30 minutes.

During all this sequence, the depolymerization plant was very stable. The condensed MMA remained below 15 °C, and the temperature in the vacuum line remained below 20 °C. From the measurement of the process parameters, there was no sign of loss of efficiency of the condenser 16.

Once the condenser entry side had cooled, the depolymerization plant was inspected with an endoscope camera. The distributor plate was clean, no trace of solid deposition, and none of the condenser tubes was plugged.

During this test, the temperature in the separator 14 measured in the inlet duct 34 initially increased to 148 °C very quickly, then to 308 °C, to finally stabilize below 335 °C. The temperature at the bottom of the separator initially increased from 81.6 °C to 94.4 °C and stabilized below 105 °C. This is a sign that the second temperature probe is in the liquid phase at the bottom of the separator 14, and that MMA is at its boiling point or of one of the azeotrope it can form with the other impurities.

The temperature at the entrance of the condenser 16 initially increased and stabilized between 250 and 277 °C.

The Methyl Methacrylate and Methanol contents were measured by Gas Chromatography using an internal standard and a calibration. They were found at 93 +/-2 wt %, and 0.4 wt % respectively (on four independent measurements during the test).

Hence, with the setup according to the invention, the unit could be operated without any problem, for several hours while increasing the productivity from 60 to 100 kg/h, and without affecting the MMA and Methanol contents in the product.

### Comparative example 3

Black Cast PMMA (also known as smokey cast PMMA) and white cast PMMA (used for thermoformed bath-tubs) were fed to the depolymerization unit. The smokey cast PMMA was fed at a feed rate of 50 kg/h using a first feeder and the white cast PMMA was fed at a feed rate of 50 kg/h using a second feeder.

The depolymerization unit is heated at 430 °C, and the Smokey cast PMMA is fed for about 1 hour, then the white cast PMMA was fed for about 1 hour and 20 minutes. The temperature of the depolymerization unit was then increased to 470 °C with operating the depolymerization unit for another 60 minutes.

There were no sign of deviation of the operating conditions of the condenser 16. Nevertheless, the condenser 16 was deconstructed, for visual inspection of the distribution plate and condenser tubes. A layer of fine gray-greenish powder accumulated in the condenser head above the distribution plate. The gas could still flow to the condenser 16 but was slowly plugging the depolymerization unit.

During the processing of the smokey cast PMMA, the temperature in the separator 14 varied between 24 and 28.8 °C. The temperature at the entrance of the condenser 16 initially increased and stabilized between 280 and 347 °C.

The Methyl Methacrylate and Methanol content were measured by Gas Chromatography using an internal standard and a calibration. It was found at 96 +/-2 wt %, and 0.2 wt % respectively.

During the processing of the white cast PMMA, the temperature in the separator 14 varied between 32 and 49.7 °C. The temperature at the entrance of the condenser 16 initially increased and stabilized between 335 and 337 °C.

The Methyl Methacrylate and Methanol content were measured by Gas Chromatography using an internal standard and a calibration. They were found at 94 +/-2 wt %, and 0.2 wt % respectively.

### Example 4 according to the invention

The depolymerization unit was fed with smokey cast PMMA and operated at 470 °C. First, the depolymerization unit was operated with a feed rate ramp up to 100 kg/h in 1 hour and then stabilized at 100 kg/h for 1 hour 30 minutes. The feed rate was then decreased to 90 kg/h and, kept in these conditions for 20 minutes, further decreased to 80 kg/h and kept in these conditions for 20 minutes, and then increased back to 90 kg/h and kept in these conditions for 5 hours.

In total 733 kg of PMMA were processed, without any sign of loss of efficiency. The pressure drop on the depolymerization unit was stable and there was no sign of loss of efficiency of the condenser 16.

During this test, the temperature in the separator 14 in the inlet duct 34 initially increased to 141 °C very quickly, then to 290 °C, to finally stabilize below 314 °C. The temperature at the bottom of the separator 14 initially increased from 110 °C to 282 °C and stabilized below 299 °C. This is a sign that the temperature probe is in the gas phase at the bottom of the separator 14, and that MMA in liquid phase does not accumulate in the bottom of the separator 14.

The temperature at the entrance of the condenser 16 initially increased and stabilized between 261 and 265 °C.

The Methyl Methacrylate and Methanol content were measured by Gas Chromatography using an internal standard and a calibration. They were found at 96 +/-2 wt %, and 0.2 wt % respectively (on 7 independent measurements during the test).

With the setup according to the invention, the unit could be operated without any problem, for several hours while increasing the feed rate from 50 to 90 kg/h, and without affecting the MMA and Methanol contents in the product.

On the following day, without intermediate cleaning of the condenser 16 nor of the separator 14, 503 kg of clear sheet extrusion material was processed in stable conditions at a feed rate of 70 kg/h, at a temperature of 470 °C and for 7h30 minutes including ramp up to stable conditions. No sign of loss of efficiency of the condenser 16 was detected.

On the following day, the depolymerization unit was operated with controlled mixtures of the very same white cast PMMA as in the comparative example 3 and the clear sheet extrusion PMMA of the previous day. The depolymerization unit operated at a feed rate of 70 kg/h and at a temperature of 470 °C.

The depolymerization unit was operated according to the following sequence: with pure white cast PMMA for about 1 hour, then with a mix of 5 wt % clear sheet extrusion PMMA and 95 wt % white cast PMMA for 1 hour, then with a mix of 10 wt % clear sheet extrusion PMMA and 90 wt % white cast PMMA for 1 hour, then with a mix of 20 wt % clear sheet extrusion PMMA and 80 wt % white cast PMMA for 1 hour, then with a mix of 30 wt % clear sheet extrusion PMMA and 70 wt % white cast PMMA for 1 hour, then with a 65 wt % clear sheet extrusion PMMA and 35 wt % white cast PMMA for 1 hour, and finally with 100 % clear sheet extrusion PMMA for 1 hour. A total of 572 kg of PMMA was processed during that sequence.

At the end, no sign of plugging of the depolymerization unit was detected. Nevertheless, the depolymerization unit was deconstructed for inspection. The condenser 16 shown a small sign of deposition of a sticky material, which contained TiO₂ which is the pigment of the white cast PMMA. The unit operated safely and stably, for a much longer time when using the design of the invention.

During this test, the temperature in the inlet duct 34 of the separator 14 initially increased very quickly, then stabilized between 250 °C and 320 °C. The temperature at the bottom of the separator 14 initially increased from 58 °C to 94 °C and stabilized below 147 °C. This is a sign that the temperature probe is initially in the liquid phase at the bottom of the separator 14, and that MMA does not accumulate in the bottom of the separator 14 and evaporates. The temperature at the entrance of the condenser 16 initially increased and stabilized between 200 and 238 °C.

The Methyl Methacrylate and Methanol content were measured by Gas Chromatography using an internal standard and a calibration. They were found at 97 +/-2 wt %, and 0.2 wt % respectively.

With the setup according to the invention, the depolymerization unit could be operated without any problem, for several hours while increasing the feed rate from 50 to 70 kg/h and with an improved MMA content in the product from 94 wt % to 97 wt %.

The depolymerization method and the corresponding depolymerization system 10 for implementing the depolymerization method allows producing a liquid monomer from a feed material containing the corresponding polymer, with obtaining a satisfying purity and maximizing the running time of the depolymerization system 10.

Indeed, providing a separator 14 allows trapping impurities that may be present in the gas stream and avoid these impurities clogging the components of the depolymerization system 10 that are located downstream the separator 14.

Operating the separator in specific temperature ranges allow avoiding any condensation and polymerization of the monomer in the separator 14. This further allows spraying cold liquid monomer inside the separator 14 for controlling the temperature of the gas stream inside the separator 14.

Some specific arrangements of the separator 14, such as the limit linear velocity of the gas stream, the separation chamber 32, the inlet duct 14 having a varying cross-section and/or extending around the separation chamber 32, the collection tube 36 with a specific structure and/or the internal demisting device 46, allow trapping the impurities efficiently while managing temperature of the gas stream and allowing the gaseous monomer to flow to the separator outlet 14B for feeding the condenser 16.

The separator is advantageous independently from the depolymerization method presented above.

Hence, the invention also relates to a separator for a depolymerization system, the separator being configured for receiving a gas stream and for removing impurities from the gas stream,

The separator comprises one or several of the following features, taken in isolation or in any technically feasible combination:
- the separator comprise a separation chamber and an inlet duct having an upstream end for receiving the gas stream and a downstream end opening into the separation chamber.
- the inlet duct is configured for progressively reducing the linear velocity of the gas stream from the upstream end towards the downstream end of the inlet duct;
- the area of the cross-section of the inlet duct increases from the upstream end to the downstream end;
- the inlet duct has a height that increases from the upstream end to the downstream end of the inlet duct and/or a width that increases from the upstream end to the downstream end of the inlet duct;
- the inlet duct is delimited by a lower surface and an upper surface, the distance between the lower surface and the upper surface increasing from the upstream end to the downstream end of the inlet duct;
- at least one of the lower surface and the upper surface extends in helix around a central axis;
- the lower surface is inclined, whereby liquid depositing on the lower surface flows to the separation chamber;
- the upper surface is inclined;
- the separator comprises an evacuation opening for evacuation of products deposited inside the separator and scrapping device configured for scraping products deposited inside the separator towards the evacuation opening;
- the evacuation opening is provided on a bottom wall of the separator;
- the inlet duct extend around the separation chamber which is at least partially surrounded by the inlet duct;
- a partition wall separates the inlet duct from the separation chamber;
- the partition wall is a portion of a cylinder;
- in view along a central axis of the separation chamber, the partition wall extends a long a portion of a circle or a portion of a spiral;
- the separator comprises a bottom part and an top part to be fitted on onto the other, the bottom par comprising a bottom and a peripheral wall extending upwardly from the periphery of the bottom wall, the peripheral wall defining an upper opening and an internal volume, the top part comprising a lid for closing an upper opening and an internal structure extending downwardly from the lid for insertion into the internal volume of the bottom part upon closing the upper opening with the lid and for defining the inlet duct and the separation chamber inside the internal volume ;
- the separator comprises a collection tube extending in the separation chamber, the collection tube comprising at least one inlet section, each inlet section comprising at least one inlet opening for gas present in the separation chamber to enter into the collection tube;
- the separator comprises baffles arranged on the collection tube, each baffle extending around a respective inlet section;
- each baffle is frustroconical with widening downwardly, each baffle having an upper edge fixed to the collection tube and a free lower edge.

## Claims

1. Depolymerization method for recovering a monomer from a polymer, the depolymerization method comprising the successive steps of:
- pyrolyzing a feed material containing the polymer into a pyrolysis reactor so as to generate a gas stream;
- directing the gas stream from the pyrolysis reactor to a separator configured for removing impurities from the gas stream;
- directing the gas stream from the separator to a condenser for condensing the monomer contained in the gas stream;
wherein the temperature of the gas stream in the separator is maintained equal or higher than the boiling temperature of the monomer.

2. Depolymerization method according to claim 1, wherein the temperature of the gas stream in the separator is lower than the boiling temperature of dimer of the monomer and/or lower than the boiling temperature of trimer of the monomer at least in a downstream section of the separator.

3. Depolymerization method according to claim 1 or 2, wherein the largest cross-section of the internal flow passage of the separator has an area that is at least 0.001 times the mass flow rate of the gas stream entering the separator in operation, the area being expressed in square meters (m²) and the mass flow rate being expressed in kilograms per hour (kg/h).

4. Depolymerization method according to any one of the preceding claims, wherein the area of the largest cross-section of the internal flow passage in the separator is at least ten times the area of the smallest cross-section in the separator.

5. Depolymerization method according to any one of the preceding claims, wherein a linear velocity of the gas stream in the separator is lower than a maximum linear velocity.

6. Depolymerization method according to claim 5, wherein the maximum linear velocity is of 0.50 m/s, in particular 0.15 m/s, still in particular of 0.10 m/s.

7. Depolymerization method according to anyone of the preceding claim, wherein the area of the largest cross-section of an internal flow passage of the separator is chosen as a function of the maximum feed rate of the pyrolysis reactor, the depolymerization temperature of the polymer and the molar weight of the polymer such that the linear velocity of the gas stream in the downstream section of the inlet duct is lower than the maximum linear velocity at the maximum feed rate of the pyrolysis reactor.

8. Depolymerization method according to any one of the preceding claims, wherein the residence time of the gas stream in the separator is comprised between 1 s and 30 s, preferably between 5 s and 25 s, still preferably between 10 s and 20 s.

9. Depolymerization method according to any one of the preceding claims, comprising spraying liquid monomer retrieved from the condenser inside the separator, the liquid monomer being sprayed into the gas stream.

10. Depolymerization method according to claim 9, wherein the cold monomer is sprayed into the separator such that the mass rate of cold monomer sprayed into the separator is equal or inferior to 93%, still preferably equal or inferior to 83%, still preferably equal of inferior to 73%, still preferably equal or inferior to 63%, still preferably equal or inferior to 53%.

11. Depolymerization method according to claim 9 or 10, wherein liquid monomer is sprayed into the separator such that the mass rate of cold monomer sprayed into the separator is comprised equal or superior to 10%, still preferably equal or superior to 20%, preferably equal or superior to 30%, still preferably equal or superior to 40%, still preferably equal or superior to 50%.

12. Depolymerization method according to anyone of the preceding claims, wherein the gas stream is slowed down progressively inside the separator.

13. Depolymerization method according to any one of the preceding claims, wherein the cross section of the internal flow passage of the separator increases progressively when considering the direction of flow of the gas stream.

14. Depolymerization method according to any one of the preceding claims, wherein the separator comprises a separation chamber extending along a central axis, the separator being configured for the gas stream to flow circularly in the separation chamber.

15. Depolymerization method according to claim 14, the separator having an inlet duct for receiving the gas stream generated by the pyrolysis reactor, the inlet duct opening into the separation chamber.

16. Depolymerization method according to claim 15, wherein the inlet duct extends around the separation chamber.

17. Depolymerization method according to anyone of the preceding claims, comprising coalescing mist droplets present in the gas stream by providing an internal demisting device inside the separator.

18. Depolymerization system configured for implementing a depolymerization method as in any one of the preceding claim for recovering a monomer from a polymer, the depolymerization system comprising :
- a pyrolysis reactor for pyrolyzing a feed material containing the polymer so as to generate a gas stream from the feed material ;
- a separator for receiving the gas stream from the pyrolysis reactor and removing impurities from the gas stream ;
- a condenser for receiving the gas stream from the separator for condensing the monomer contained in the gas stream;
wherein the depolymerization system is configured such that in operation the temperature of the gas stream in the separator is maintained equal to or higher than the boiling temperature of the monomer.
